# EUROPEAN PATENT APPLICATION

(11) **EP 4 649 941 A1**
(43) Date of publication of application: **19.11.2025**
(21) Application number: 23915840.5
(22) Date of filing: 29.12.2023
(51) Int. Cl.: A61K 9/06, A61K 47/36, A61K 31/381, A61L 2/02

(54) **METHOD FOR PREPARING STERILE IN-SITU GEL ON BASIS OF SOLVENT REMOVAL TECHNIQUE AND PRODUCT THEREOF**

(30) Priority: 10.01.2023 CN 202310035221
(71) Applicant: Sichuan Kelun Pharmaceutical Research Institute Co., Ltd., Chengdu, Sichuan 611138 (CN); Hunan Kelun Pharmaceutical Research Co., Ltd., Yueyang, Hunan 414000 (CN)
(72) Inventor: ZHAO, Jinlong, Chengdu, Sichuan 611138 (CN); SU, Zhengxing, Chengdu, Sichuan 611138 (CN); YANG, Yifan, Chengdu, Sichuan 611138 (CN); XIE, Jia, Chengdu, Sichuan 611138 (CN); LI, Ming, Chengdu, Sichuan 611138 (CN); BAO, Fei, Chengdu, Sichuan 611138 (CN); ZHAO, Dong, Chengdu, Sichuan 611138 (CN); LIU, Sichuan, Chengdu, Sichuan 611138 (CN)
(74) Representative: Ipsilon
(86) International application number: PCT/CN2023/143080
(87) International publication number: WO 2024/149087

(57) **Abstract**

A method for preparing a sterile in-situ gel on the basis of a solvent removal technique and a product thereof. Provided is a method for preparing an in-situ gel. Compared with traditional sterile production methods for in-situ gel preparations, the viscosity of a preparation solution is reduced by means of adding a volatile solvent in the production process, so that filtering and sterilization can be performed, and subsequently, the volatile solvent in the preparation can be removed by means of various methods, which does not have any adverse effect on the finally prepared in-situ gel preparation. Research data shows that for the in-situ gel preparation prepared by means of using the preparation method, volatile solvent residues therein meet safety standards, and compared with preparations prepared by the traditional preparation method of radiation sterilization, the types and amounts of degradation impurities of the polymer and pharmaceutically active ingredients therein are reduced, so that the safety of the preparation is improved.

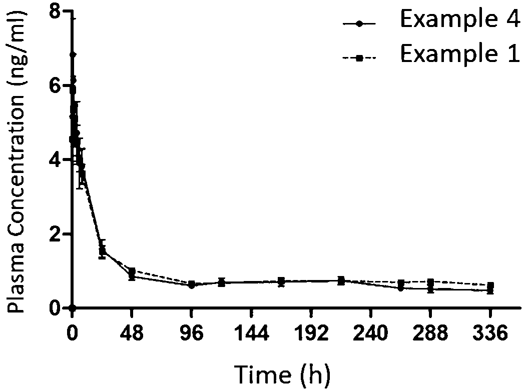

## Description

### CROSS-REFERENCE TO RELATED APPLICATION

The present disclosure claims the priority to and the benefit of Chinese Patent Application No. 202310035221.1 filed with the Patent Office of CNIPA on January 10, 2023 and entitled "METHOD FOR PREPARING STERILE IN-SITU GEL ON BASIS OF SOLVENT REMOVAL TECHNIQUE AND PRODUCT THEREOF", the entire contents of which are incorporated herein by reference.

### TECHNICAL FIELD

The present disclosure belongs to the field of pharmaceutical formulations, and specifically relates to a method for preparing a sterile in-situ gel on the basis of a solvent removal technique and a sterile in-situ gel prepared by the same method.

### BACKGROUND

In situ forming implant (ISFI) is a novel drug delivery system composed of a solution or semi-solid mixture of biodegradable polymer, which is capable of being solidified/gelated at the introduction site to form a depot. Depending on the composition of formulation, ISFIs are primarily classified by different mechanisms of formation such as in situ precipitation, organic gelation or polymer crosslinking. Of these, the Atrigel^{®} system, based on solvent diffusion-induced in situ precipitation, is currently market-oriented and the most applied technology with several commercial products having been put on the market, and is widely used in the fields such as treatment of periodontitis (Atridox^{®}), advanced prostate cancer (Eligard^{®}), opioid use disorder (Subloade^{®}), and schizophrenia (Perseris^{®}).

Dunn *et al.* first put forward the concept related to the Atrigel^{®} technology in 1987. This technology is intended mainly to mix a biodegradable polymer carrier (e.g., poly(lactic-co-glycolic) (PLGA) or polylactic acid (PLA)) with an organic solvent (e.g., N-methyl pyrrolidone (NMP)) till dissolved to form a carrier system, and then dissolve or disperse a small molecular, polypeptide or macromolecular drug or the like therein to form a solution or suspension introducible into the body. Under long-term high temperature conditions, the polymer carrier (such as PLGA) and the active pharmaceutical ingredient in such an in-situ gel formulation are prone to degradation, and interactions therebetween may occur, so it is impossible to perform terminal sterilization by dry heat or moist heat. Furthermore, as a result of a higher polymer concentration and too high gel viscosity, it is also difficult for such an in-situ gel formulation to satisfy the sterilization requirements for formulations introduced into the body by means of filtration sterilization. In view of the foregoing, such in-situ gel formulations are typically sterilized by means of irradiation predominated by γ-ray irradiation around the world.

Irradiation sterilization follows the principle below: atoms drop from a high-energy state to a low-energy state and release high-energy rays, these rays have high energy and strong penetrability, which can cause microorganisms in drugs to undergo a series of biophysical and biochemical reactions, and inhibit and destroy the metabolism and the growth and development of the microorganisms, thereby achieving the effect of killing the microorganisms, but can also accelerate degradation of the polymer carrier and the active pharmaceutical ingredient in the in-situ gel and promote the interactions between the polymer and the drug, resulting in some undesirable impurities. Therefore, the carrier component and the drug component of such formulation are required to be packaged separately in general, and then formulated immediately before use, which quite probably brings inaccurate dosage caused by improper blending or use for medical staff during application. Additionally, since irradiation-related factories need to go through rigorous appraisal for certification and site selection, it is usually necessary for pharmaceutical manufacturers to entrust professional irradiation factories to carry out the irradiation sterilization process. But none of the stages from the completion of product packaging to irradiation via transportation is carried out in accordance with the GMP or cGMP requirements, which poses high risks in management and quality assurance.

Therefore, it is of great significance to develop a simple aseptic preparation method of in-situ gel formulations.

### SUMMARY

### Technical Problem

In view of the problems existing in the prior art, for example, the available aseptic production methods for in-situ gel formulations accelerate the degradation of drugs and polymer carriers, result in additional impurities and cause inconvenience in use, the present disclosure provides a preparation method of an in-situ gel formulation, which resolves the problems existing in conventional aseptic production methods for in-situ gel formulations by introducing the followings into the conventional process for preparing in-situ gels: the addition of a volatile solvent for reducing the viscosity of the gel solution so as to enable the filtration sterilization and the removal of the volatile solvent through a subsequent operation.

### Solution to Problem

In the first aspect, the present disclosure provides a preparation method of an in-situ gel formulation, the preparation method comprising the steps of:
i: mixing a biodegradable polymer and a volatile solvent among raw materials for preparation, optionally with part or all of components among the remaining raw materials for preparation, until homogeneous to obtain a feed liquid I;
ii: sterilizing the feed liquid I by filtration to obtain a sterile feed liquid I;
iii: removing the volatile solvent from the sterile feed liquid I; and
when the feed liquid I does not contain all of the raw materials for preparation, the preparation method further comprising the step of:
iv: mixing a product obtained in iii with the raw material(s) not contained in the feed liquid I which is(are) sterile until homogeneous under sterile conditions;
wherein an active pharmaceutical ingredient, a non-volatile solvent, and an optional release modifier are also among the raw materials for preparation.

Further, when the feed liquid I does not contain all of the raw materials for preparation, the product obtained in iii is mixed with the raw material(s) not contained in the feed liquid I until homogeneous in iv by any one or more of the following methods (a) to (g):
(a) adding the active pharmaceutical ingredient which is sterile through a sterile feeding device;
(b) adding a solution of the active pharmaceutical ingredient and the non-volatile solvent after filtration sterilization;
(c) adding a solution of the active pharmaceutical ingredient and the release modifier, if present, after filtration sterilization;
(d) adding a solution of the active pharmaceutical ingredient, the non-volatile solvent, and the release modifier, if present, after filtration sterilization;
(e) adding the non-volatile solvent after filtration sterilization;
(f) adding a solution of the non-volatile solvent and the release modifier, if present, after filtration sterilization; and
(g) adding the release modifier, if present, after filtration sterilization.

Preferably, when the feed liquid I does not contain all of the raw materials for preparation, the feed liquid I satisfies any one of the following conditions (j) to (p):
(j) the feed liquid I contains the active pharmaceutical ingredient and the non-volatile solvent and does not contain the release modifier;
(k) the feed liquid I contains the active pharmaceutical ingredient and the release modifier and does not contain the non-volatile solvent;
(l) the feed liquid I contains the active pharmaceutical ingredient and does not contain the non-volatile solvent or the release modifier;
(m) the feed liquid I contains the non-volatile solvent and the release modifier and does not contain the active pharmaceutical ingredient;
(n) the feed liquid I contains the non-volatile solvent and does not contain the active pharmaceutical ingredient or the release modifier;
(o) the feed liquid I contains the release modifier and does not contain the active pharmaceutical ingredient or the non-volatile solvent; and
(p) the feed liquid I does not contain the active pharmaceutical ingredient, the non-volatile solvent, or the release modifier.

Further, the volatile solvent is a volatile alkane, a volatile halogenated alkane, a volatile alcohol, a volatile ketone, a volatile ester, a volatile organic cyanide, or a volatile ether.

Preferably, among the raw materials for preparation, the mass ratio of the volatile solvent to the biodegradable polymer varies from 10:1 to 1:1.

Furthermore, the volatile solvent is dichloromethane, trichloromethane, or acetone.

Further, in the step iii, a way to remove the volatile solvent from the sterile feed liquid I is volatilization under one or more conditions selected from decompression, temperature control, ventilation, and stirring.

Further, the biodegradable polymer is a polyester, a polyester copolymer, or a block copolymer.

Furthermore, the biodegradable polymer is PLGA, PLA, PCL, POE, PEG-PLA, PLA-PEG-PLA, PEG-PLGA, PEG-PLGA-PEG, TPGS-PLGA, or TEG-POE.

Further, the non-volatile solvent is N-methyl pyrrolidone and/or dimethyl sulfoxide.

Further, the optional release modifier is one or more selected from ethyl acetate, medium-chain triglyceride(s), glyceryl triacetate, glyceryl tricaprylate, benzyl benzoate, and benzyl alcohol.

Preferably, the optional release modifier is benzyl benzoate and/or glyceryl triacetate.

In the second aspect, the present disclosure provides an in-situ gel formulation obtained by the preparation method according to the first aspect of the present disclosure.

### Advantageous Effects of the Invention

The present disclosure provides a preparation method of an in-situ gel. Compared with the conventional aseptic production methods for in-situ gel formulations, the preparation method in the present disclosure reduces the viscosity of the solution for formulation by adding a volatile solvent in the production process, so as to enable the filtration sterilization, and the volatile solvent can be removed from the formulation by multiple methods, thus avoiding any adverse effect on the final in-situ gel formulation.

Research data show that the in-situ gel formulation obtained by the preparation method provided herein contains volatile solvent residues that meet the safety standards, and contains both less type and less content of impurities resulting from degradation of the polymer and the active pharmaceutical ingredient as compared to the formulations obtained by conventional preparation methods through irradiation sterilization, thereby improving the safety of the formulation.

Meanwhile, the sterile in-situ gel formulation obtained by the preparation method provided herein has already contained the active pharmaceutical ingredient, which solves the problems of inconvenience in use and inaccurate dosage caused by separate packaging of the carrier and the active pharmaceutical ingredient in the prior art, and greatly improves the convenience and accuracy of the formulation at the application level.

In addition, the preparation method of the in-situ gel formulation provided herein is simple and convenient to operate, and allows pharmaceutical manufacturers to independently produce sterile pharmaceuticals while ensuring product quality, which is more conducive to the management and the quality assurance of the production process. Besides, the preparation method of the in-situ gel formulation provided herein has low quality requirements for sterile raw materials and sterile feeding space and equipment, and saves production cost.

### BRIEF DESCRIPTION OF THE DRAWINGS

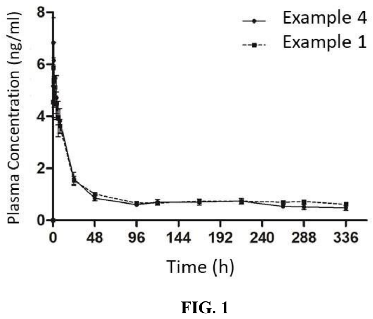

FIG. 1 shows plasma concentration vs. time curves of in-situ gel formulations of rotigotine of Example 1 and Example 4 in rats.

### DETAILED DESCRIPTION

The embodiments of the present disclosure will be described below, but the present disclosure is not limited thereto. The present disclosure is not limited to the elements described below. Various modifications may be made within the scope as claimed in the invention. Moreover, the embodiments or examples obtained by appropriately combining the technical means disclosed respectively in different embodiments or examples are also encompassed in the technical scope of the present disclosure.

### <Definitions>

The numerical range represented by "numerical value A to numerical value B" or "numerical value A - numerical value B" used in the present disclosure refers to the range including the endpoint values A and B.

The term "may" used in the present disclosure involves both the meaning of doing something and the meaning of not doing something.

In the present disclosure, the term "optional" or "optionally" means that the event or case described subsequently may or may not occur, and the description includes the case where the event occurs and the case where the event does not occur.

In the present disclosure, the term "a" or "an" or "the" may refer to "one", or refer to "one or more", "at least one", and "one or more than one".

In the present disclosure, the term "about" may indicate that a value includes the standard deviation of an error caused by the device or method used to measure the value. The numerical ranges and parameters used to define the present disclosure are all approximate numerical values, and the numerical values involved in the specific examples have been presented herein as accurately as possible. However, any numerical value inevitably contains a standard deviation caused by the aforementioned testing device or method in nature. Therefore, it should be understood that all the ranges, quantities, numerical values, and percentages used herein have already been modified by "about" unless otherwise expressly stated. The term "about" used herein generally means that the actual value is within ±10%, ±5%, ±1%, or ±0.5% of a particular numerical value or range.

The parameters for PLGA used herein are explained as follows: taking PLGA (7525 DLG 2A) as an example, "7525" means that the molar ratio of lactide to glycolide is 75:25; "DLG" means lactide/glycolide copolymer, and if represented as "DL", it means polylactide; "2" acts as a sign for intrinsic viscosity, where 1 means that the intrinsic viscosity is 0.05 to 0.15 dL/g, 1.5 means that the intrinsic viscosity is 0.10 to 0.20 dL/g, 2 means that the intrinsic viscosity is 0.15 to 0.25 dL/g, 2.5 means that the intrinsic viscosity is 0.20 to 0.30 dL/g, 3 means that the intrinsic viscosity is 0.25 to 0.35 dL/g, 3.5 means that the intrinsic viscosity is 0.30 to 0.40 dL/g, 4 means that the intrinsic viscosity is 0.35 to 0.45 dL/g, 4.5 means that the intrinsic viscosity is 0.40 to 0.50 dL/g, 5 means that the intrinsic viscosity is 0.45 to 0.55 dL/g, 6 means that the intrinsic viscosity is 0.50 to 0.70 dL/g, 7 means that the intrinsic viscosity is 0.60 to 0.80 dL/g, 8 means that the intrinsic viscosity is 0.70 to 0.90 dL/g, and 9 means that the intrinsic viscosity is 0.80 to 1.00 dL/g; the PLGA having a molecular weight of 100000 Da has an intrinsic viscosity of 1 dL/g; and "A" means that the polymer is end-capped with carboxylic acid groups, and if represented as "E", it means that the polymer is end-capped with carboxylic ester groups.

In the present disclosure, the "water" includes any water usable in the art, such as deionized water, distilled water, ion exchange water, double distilled water, high-purity water, and purified water.

In the present disclosure, the "normal temperature" refers to 25±2°C.

In the present disclosure, the "normal pressure" refers to one standard atmospheric pressure.

Additionally, unless otherwise defined, the other technical and scientific terms used herein have the same meanings as typically understood by one of ordinary skill in the art to which the present disclosure belongs.

The present disclosure is accomplished mainly on the basis of the insights of:
In order to develop a simple aseptic preparation method of in-situ gels, the present inventors have conducted intensive studies and provided a method for preparing sterile in-situ gels based on a solvent removal technique, that is, during conventional preparation process of in-situ gels, adding a volatile solvent (such as trichloromethane, dichloromethane or acetone) to reduce the viscosity of the gel solution, then sterilizing by filtration through a 0.22-µm filter membrane, subsequently removing the volatile solvent, and finally filling in a sterile environment.

The technical solutions of the present disclosure are further illustrated in detail below.

### <Preparation Method of In-Situ Gel Formulation>

The present disclosure provides a preparation method of an in-situ gel formulation, the method comprising the steps of:
i: mixing a biodegradable polymer and a volatile solvent among raw materials for preparation, optionally with part or all of components among the remaining raw materials for preparation, until homogeneous to obtain a feed liquid I;
ii: sterilizing the feed liquid I by filtration to obtain a sterile feed liquid I;
iii: removing the volatile solvent from the sterile feed liquid I; and
when the feed liquid I does not contain all of the raw materials for preparation, the preparation method further comprising the step of:
iv: mixing a product obtained in iii with the raw material(s) not contained in the feed liquid I which is(are) sterile until homogeneous under sterile conditions;
wherein an active pharmaceutical ingredient, a non-volatile solvent, and an optional release modifier are also among the raw materials for preparation.

### [Raw materials for preparation for In-Situ Gel Formulation]

The raw materials for preparation according to the present disclosure include a volatile solvent, a biodegradable polymer, a non-volatile solvent, an active pharmaceutical ingredient, and an optional release modifier.

In some embodiments, the raw materials for preparation according to the present disclosure include a volatile solvent, a biodegradable polymer, a non-volatile solvent, and an active pharmaceutical ingredient.

In other embodiments, the raw materials for preparation according to the present disclosure include a volatile solvent, a biodegradable polymer, a non-volatile solvent, an active pharmaceutical ingredient, and a release modifier.

### Volatile Solvent

In the present disclosure, the "volatile solvent" refers to a liquid substance having a volatile property at normal temperature and normal pressure. The specific type of the volatile solvent is not particularly limited in the present disclosure and may be determined by a person skilled in the art as actually required.

In some embodiments, the volatile solvent according to the present disclosure is a volatile halogenated alkane, a volatile alcohol, a volatile ketone or a volatile ether. Suitable volatile alkane includes, but is not limited to, pentane, cyclopentane, cyclohexane, n-hexane, n-octane, isooctane, heptane, and so forth. Suitable volatile halogenated alkane includes, but is not limited to, dichloromethane, trichloromethane, carbon tetrachloride, and so forth. Suitable volatile alcohol includes, but is not limited to, methanol, ethanol, propanol, butanol, and so forth. Suitable volatile ketone includes, but is not limited to, acetone, cyclohexanone, and so forth. Suitable volatile ester includes, but is not limited to, ethyl acetate and so forth. Suitable volatile organic cyanide includes, but is not limited to, acetonitrile and so forth. Suitable volatile ether includes, but is not limited to, diethyl ether.

In some embodiments, the boiling point of the volatile solvent according to the present disclosure is lower than 150°C, preferably lower than 100°C, more preferably lower than 70°C.

In some specific embodiments, the volatile solvent according to the present disclosure is dichloromethane, trichloromethane or acetone. The above three volatile solvents have a relatively low boiling point, where dichloromethane has a boiling point of about 39°C, trichloromethane has a boiling point of about 61°C, and acetone has a boiling point of about 53°C, which greatly differ from the boiling point (about 202°C to 204°C) of N-methyl pyrrolidone as a non-volatile solvent in the formulation, and contribute to removal of the volatile solvent without volatilizing the non-volatile solvent. Moreover, all of the above three volatile solvents can dissolve the biodegradable polymer (e.g., PLGA) well, and can effectively reduce the viscosity of the biodegradable polymer to facilitate the filtration sterilization operation.

The amount of the volatile solvent is not particularly limited in the present disclosure, and may be adjusted according to the actual requirements for production only as long as the viscosity of the solution containing the biodegradable polymer could be reduced so as to allow for the filtration sterilization.

In some embodiments, the mass ratio of the volatile solvent to the biodegradable polymer varies from 10:1 to 1:1, preferably 5:1 to 1:1, for example, 5:1, 4:1, 3:1, 2:1, 1:1 or any numerical value within the range. The amount of the volatile solvent within the above range can not only satisfy the requirement for reducing the viscosity of the solution, but also contribute to the subsequent removal of the volatile solvent.

### Biodegradable Polymer

In the present disclosure, "biodegradable polymer", "bioabsorbable polymer", and "absorbable polymer" may be used interchangeably and refer to polymers that may break down by chemical or physical methods when interacting with a physiological environment, for example, the polymers are eroded, decomposed, or dissolved at the implantation site in a subject over a period of time, e.g. within several days, several weeks, or several months. Biodegradable polymers serve a temporary function in a subject, e.g. delivering bio-activators such as drugs. Biodegradable polymers may be degraded into fragments and metabolized or excreted by the host. The specific type of the biodegradable polymer is not particularly limited in the present disclosure and may be determined by a person skilled in the art as actually required.

In some embodiments, the biodegradable polymer according to the present disclosure is a polyester or a polyester copolymer. The polyester refers to a polymer in which all or substantially all of the repeating units are linked together by ester groups. The polyester may be formed by subjecting a monomer having carboxyl group and a monomer having hydroxyl group to reaction in order to form ester group. Suitable polyester includes, but is not limited to, polyglycolide, polylactide, polycaprolactone, and poly(orthoester), etc. Suitable polyester copolymer includes, but is not limited to, lactide/glycolide copolymer, ε-caprolactone/glycolide copolymer, lactide/trimethylene carbonate copolymer, lactide/glycolide/caprolactone terpolymer, lactide/glycolide/trimethylene carbonate terpolymer, lactide/caprolactone/trimethylene carbonate terpolymer, glycolide/caprolactone/trimethylene carbonate terpolymer, and lactide/glycolide/caprolactone/trimethylene carbonate tetrapolymer. In other embodiments, the biodegradable polymer according to the present disclosure may be a block copolymer. Exemplarily, it may be a PLGA- and/or PLA-based block copolymer, e.g., PEG-PLA, PLA-PEG-PLA, PEG-PLGA, PEG-PLGA-PEG, and TPGS-PLGA, or may also be a poly(orthoester)-based block copolymer, e.g., TEG-POE.

In some specific embodiments, the biodegradable polymer according to the present disclosure is lactide/glycolide copolymer, polycaprolactone or poly(orthoester).

In some more specific embodiments, the biodegradable polymer according to the present disclosure is lactide/glycolide copolymer. The molar ratio of lactide to glycolide in the lactide/glycolide copolymer is not particularly limited in the present disclosure and may be determined by a person skilled in the art as actually required. Exemplarily, the molar ratio of lactide to glycolide in the copolymer of lactide and glycolide is 50:50 to 95:5; for example, it may specifically be 50:50, 75:25, 85:15, 95:5, etc.

The molecular weight of the biodegradable polymer is not particularly limited in the present disclosure and may be determined by a person skilled in the art as actually required. Exemplarily, the molecular weight of the biodegradable polymer according to the present disclosure may be 5000 to 70000 Da, for example, 5000 Da, 10000 Da, 15000 Da, 20000 Da, 25000 Da, 30000 Da, 35000 Da, 40000 Da, 45000 Da, 50000 Da, 55000 Da, 60000 Da, 65000 Da, 70000 Da or any numerical value within the range, etc.

### Non-Volatile Solvent

In the present disclosure, the "non-volatile solvent" does not have a volatile property at normal temperature and normal pressure, and can diffuse in living organisms, be metabolized or absorbed by living organisms. The specific type of the non-volatile solvent is not particularly limited in the present disclosure and may be determined by a person skilled in the art as actually required.

In some embodiments, the non-volatile solvent according to the present disclosure is N-methyl pyrrolidone and/or dimethyl sulfoxide.

In some specific embodiments, the non-volatile solvent according to the present disclosure is N-methyl pyrrolidone.

### Release Modifier

In the present disclosure, the "release modifier" is used to regulate the *in vivo* and *in vitro* release characteristics of the active pharmaceutical ingredient in the in-situ gel formulation over time. The specific type of the release modifier is not particularly limited in the present disclosure and may be determined by a person skilled in the art as actually required.

In some embodiments, the release modifier according to the present disclosure is one or more selected from ethyl acetate, medium-chain triglyceride(s), glyceryl triacetate, glyceryl tricaprylate, benzyl benzoate, and benzyl alcohol.

In some specific embodiments, the release modifier according to the present disclosure is benzyl benzoate and/or glyceryl triacetate.

In some more specific embodiments, the release modifier according to the present disclosure is benzyl benzoate.

### Active pharmaceutical ingredient

The specific type of the active pharmaceutical ingredient in the in-situ gel formulation is not particularly limited in the present disclosure and may be selected by a person skilled in the art as actually required.

In some embodiments, the active pharmaceutical ingredient according to the present disclosure is a poorly soluble drug.

In some embodiments, the active pharmaceutical ingredient according to the present disclosure is rotigotine or pramipexole dihydrochloride.

### [Preparation Method of In-Situ Gel]

The preparation method of in-situ gels provided herein is based on the solvent removal technique, in which a volatile solvent is added in the preparation process to reduce the viscosity of the solution containing a biodegradable polymer, so that it is possible to sterilize by means of filtration, and the volatile solvent is removed from the formulation by taking advantage of its volatile property, thereby improving the convenience for preparation of sterile in-situ gel formulations.

In some embodiments, the preparation method of an in-situ gel formulation provided herein comprises the steps of:
i: mixing a biodegradable polymer and a volatile solvent among raw materials for preparation with all of components among the remaining raw materials for preparation until homogeneous to obtain a feed liquid I;
ii: sterilizing the feed liquid I by filtration to obtain a sterile feed liquid I; and
iii: removing the volatile solvent from the sterile feed liquid I.

In some specific embodiments, the preparation method of an in-situ gel formulation provided herein comprises the steps of:
i: mixing a biodegradable polymer and a volatile solvent with an active pharmaceutical ingredient, a non-volatile solvent, and an optional release modifier among raw materials for preparation until homogeneous to obtain a feed liquid I;
ii: sterilizing the feed liquid I by filtration to obtain a sterile feed liquid I; and
iii: removing the volatile solvent from the sterile feed liquid I.

In the above solution, all the raw materials can be sterilized by filtration at one time, which greatly improves the convenience in preparation of formulations. Furthermore, the experimental data substantiate that the above solution does not have any adverse impact on the active pharmaceutical ingredient and its pharmacokinetics behaviors, and the types and quantities of impurities generated in the preparation process are greatly reduced as compared to the irradiation sterilization.

In other embodiments, the preparation method of an in-situ gel formulation provided herein comprises the steps of:
i: mixing a biodegradable polymer and a volatile solvent among raw materials for preparation, optionally with part of components among the remaining raw materials for preparation, until homogeneous to obtain a feed liquid I;
ii: sterilizing the feed liquid I by filtration to obtain a sterile feed liquid I;
iii: removing the volatile solvent from the sterile feed liquid I; and
iv: mixing a product obtained in iii with the raw material(s) not contained in the feed liquid I which is(are) sterile until homogeneous under sterile conditions.

In some specific embodiments, when the feed liquid I does not contain all of the raw materials for preparation, the feed liquid I satisfies any one of the following conditions (j) to (p):
(j) the feed liquid I contains the active pharmaceutical ingredient and the non-volatile solvent and does not contain the optional release modifier;
(k) the feed liquid I contains the active pharmaceutical ingredient and the release modifier and does not contain the non-volatile solvent;
(l) the feed liquid I contains the active pharmaceutical ingredient and does not contain the non-volatile solvent or the release modifier;
(m) the feed liquid I contains the non-volatile solvent and the release modifier and does not contain the active pharmaceutical ingredient;
(n) the feed liquid I contains the non-volatile solvent and does not contain the active pharmaceutical ingredient or the release modifier;
(o) the feed liquid I contains the release modifier and does not contain the active pharmaceutical ingredient or the non-volatile solvent; and
(p) the feed liquid I does not contain the active pharmaceutical ingredient, the non-volatile solvent, or the release modifier.

For step iv, when all the raw material(s) for preparation not contained in the feed liquid I is(are) solid ingredient(s), the raw material(s) for preparation not contained in the feed liquid I which is(are) sterile may be added to the product obtained in iii using equipment such as a sterile feeding device and mixed until homogeneous. When all the raw material(s) for preparation not contained in the feed liquid I is(are) liquid ingredient(s), the raw material(s) for preparation not contained in the feed liquid I is(are) preferably sterilized by means of filtration sterilization, and the sterile component(s) that has(have) been sterilized is(are) added to the product obtain in iii and mixed until homogeneous. When the raw materials for preparation not contained in the feed liquid I include both solid ingredient(s) and liquid ingredient(s), the sterile solid ingredient(s) may be added to the product obtained in iii using equipment such as a sterile feeding device and mixed until homogeneous, and the liquid ingredient(s) is(are) sterilized by means of filtration sterilization and the sterile liquid ingredient(s) that has(have) been sterilized is(are) added to the product obtain in iii and mixed until homogeneous, or the solid ingredient(s) and the liquid ingredient(s) are mixed until homogeneous, then sterilized by filtration, and subsequently the sterile components that have been sterilized are added to the product obtain in iii and mixed until homogeneous.

In some preferred embodiments, when the feed liquid I does not contain all of the raw materials for preparation, the raw material(s) not contained in the feed liquid I is(are) mixed with the product obtained in iii until homogeneous in iv by any one or more of the methods set forth in the following methods (a) to (g):
(a) adding the active pharmaceutical ingredient which is sterile through a sterile feeding device;
(b) adding a solution of the active pharmaceutical ingredient and the non-volatile solvent after filtration sterilization;
(c) adding a solution of the active pharmaceutical ingredient and the release modifier, if present, after filtration sterilization;
(d) adding a solution of the active pharmaceutical ingredient, the non-volatile solvent, and the release modifier, if present, after filtration sterilization;
(e) adding the non-volatile solvent after filtration sterilization;
(f) adding a solution of the non-volatile solvent and the release modifier, if present, after filtration sterilization; and
(g) adding the release modifier, if present, after filtration sterilization.

In some specific embodiments, the preparation method of an in-situ gel formulation provided herein comprises the steps of:
i: mixing a biodegradable polymer and a volatile solvent with an active pharmaceutical ingredient and a non-volatile solvent among raw materials for preparation until homogeneous to obtain a feed liquid I;
ii: sterilizing the feed liquid I by filtration to obtain a sterile feed liquid I;
iii: removing the volatile solvent from the sterile feed liquid I; and
when a release modifier is among the raw materials for preparation, the preparation method further comprises the step of:
iv: mixing the product obtained in iii with a release modifier that has been sterilized by filtration until homogeneous under sterile conditions.

In some specific embodiments, the preparation method of an in-situ gel formulation provided herein comprises the steps of:
i: mixing a biodegradable polymer and a volatile solvent with an active pharmaceutical ingredient and a release modifier among raw materials for preparation until homogeneous to obtain a feed liquid I;
ii: sterilizing the feed liquid I by filtration to obtain a sterile feed liquid I;
iii: removing the volatile solvent from the sterile feed liquid I; and
the preparation method further comprises the step of:
iv: mixing the product obtained in iii with a non-volatile solvent that has been sterilized by filtration until homogeneous under sterile conditions.

In some specific embodiments, the preparation method of an in-situ gel formulation provided herein comprises the steps of:
i: mixing a biodegradable polymer and a volatile solvent with an active pharmaceutical ingredient among raw materials for preparation until homogeneous to obtain a feed liquid I;
ii: sterilizing the feed liquid I by filtration to obtain a sterile feed liquid I;
iii: removing the volatile solvent from the sterile feed liquid I; and
when a release modifier is not among the raw materials for preparation, the preparation method further comprises the step of:
iv: mixing the product obtained in iii with a non-volatile solvent that has been sterilized by filtration until homogeneous under sterile conditions.

In some specific embodiments, the preparation method of an in-situ gel formulation provided herein comprises the steps of:
i: mixing a biodegradable polymer and a volatile solvent with an active pharmaceutical ingredient among raw materials for preparation until homogeneous to obtain a feed liquid I;
ii: sterilizing the feed liquid I by filtration to obtain a sterile feed liquid I;
iii: removing the volatile solvent from the sterile feed liquid I; and
when a release modifier is among the raw materials for preparation, the preparation method further comprises the step of:
iv: mixing a non-volatile solvent with the release modifier until homogeneous, sterilizing by filtration, and then mixing with the product obtained in iii until homogeneous under sterile conditions.

In some specific embodiments, the preparation method of an in-situ gel formulation provided herein comprises the steps of:
i: mixing a biodegradable polymer and a volatile solvent with a non-volatile solvent and a release modifier among raw materials for preparation until homogeneous to obtain a feed liquid I;
ii: sterilizing the feed liquid I by filtration to obtain a sterile feed liquid I;
iii: removing the volatile solvent from the sterile feed liquid I; and
the preparation method further comprises the step of:
iv: adding a sterile active pharmaceutical ingredient to the product obtained in iii via a sterile feeding device and mixing until homogeneous.

In some specific embodiments, the preparation method of an in-situ gel formulation provided herein comprises the steps of:
i: mixing a biodegradable polymer and a volatile solvent with a non-volatile solvent and a release modifier among raw materials for preparation until homogeneous to obtain a feed liquid I;
ii: sterilizing the feed liquid I by filtration to obtain a sterile feed liquid I;
iii: removing the volatile solvent from the sterile feed liquid I; and
the preparation method further comprises the step of:
iv: mixing an active pharmaceutical ingredient with the non-volatile solvent until homogeneous, sterilizing by filtration, and then mixing with the product obtained in iii until homogeneous under sterile conditions.

In some specific embodiments, the preparation method of an in-situ gel formulation provided herein comprises the steps of:
i: mixing a biodegradable polymer and a volatile solvent with a non-volatile solvent among raw materials for preparation until homogeneous to obtain a feed liquid I;
ii: sterilizing the feed liquid I by filtration to obtain a sterile feed liquid I;
iii: removing the volatile solvent from the sterile feed liquid I; and
when a release modifier is not among the raw materials for preparation, the preparation method further comprises the step of:
iv: adding a sterile active pharmaceutical ingredient to the product obtained in iii via a sterile feeding device, and mixing until homogeneous.

In some specific embodiments, the preparation method of an in-situ gel formulation provided herein comprises the steps of:
i: mixing a biodegradable polymer and a volatile solvent with a non-volatile solvent among raw materials for preparation until homogeneous to obtain a feed liquid I;
ii: sterilizing the feed liquid I by filtration to obtain a sterile feed liquid I;
iii: removing the volatile solvent from the sterile feed liquid I; and
when a release modifier is among the raw materials for preparation, the preparation method further comprises the step of:
iv: mixing an active pharmaceutical ingredient with the release modifier until homogeneous, sterilizing by filtration, and then mixing with the product obtained in iii until homogeneous under sterile conditions.

In some specific embodiments, the preparation method of an in-situ gel formulation provided herein comprises the steps of:
i: mixing a biodegradable polymer and a volatile solvent with a release modifier among raw materials for preparation until homogeneous to obtain a feed liquid I;
ii: sterilizing the feed liquid I by filtration to obtain a sterile feed liquid I;
iii: removing the volatile solvent from the sterile feed liquid I; and
the preparation method further comprises the step of:
iv: mixing an active pharmaceutical ingredient with a non-volatile solvent until homogeneous, sterilizing by filtration, and then mixing with the product obtained in iii until homogeneous under sterile conditions.

In some specific embodiments, the preparation method of an in-situ gel formulation provided herein comprises the steps of:
i: mixing a biodegradable polymer and a volatile solvent among raw materials for preparation until homogeneous to obtain a feed liquid I;
ii: sterilizing the feed liquid I by filtration to obtain a sterile feed liquid I;
iii: removing the volatile solvent from the sterile feed liquid I; and
the preparation method further comprises the step of:
iv: mixing an active pharmaceutical ingredient, a non-volatile solvent, and an optional release modifier until homogeneous, sterilizing by filtration, and then mixing with the product obtained in iii until homogeneous under sterile conditions.

For the filtration sterilization operation in the preparation method of in-situ gel formulations provided herein, it is preferable to filter through a microporous filter membrane, more preferably through a 0.22-µm filter membrane.

The way to remove the volatile solvent in the preparation method of in-situ gel formulations is not particularly limited in the present disclosure and may be selected by a person skilled in the art as actually required. In some embodiments, the way to remove the volatile solvent according to the present disclosure is volatilization under one or more conditions selected from decompression, temperature control, ventilation, and stirring. Furthermore, the volatile solvent may be removed using appropriate equipment. Exemplarily, the equipment includes, but is not limited to, a thin film evaporator, a rotary evaporator or an air blowing device, etc.

After the steps included in the preparation method of in-situ gel formulations provided herein, there may be further included a step of adding additional ingredients and may be further included a step of filling, etc.; preferably, the additional ingredients are sterile; preferably, the additional ingredients are not an active pharmaceutical ingredient.

### Examples

To express the technical solution of the present disclosure more clearly, it will be further described below with reference to specific examples, which cannot be used to limit the scope of the present disclosure but serve only as part of examples of the present disclosure. Unless otherwise stated, all of the instruments, reagents, materials, experimental animals, etc. used for or involved in the present disclosure are commercially available through conventional means. Where the specific conditions are not indicated in the examples, conventional conditions or the conditions recommended by manufacturers shall prevail.

### Example 1 (Comparative Example)

33.75 g of PLGA (7525 DLG 2A), 28.9 g of NMP, 2.75 g of rotigotine, and 12.4 g of benzyl benzoate were precisely weighed, mixed, dissolved while stirring until homogeneous, and subpackaged into 1-mL pre-filled syringes. They were then sterilized by 25 kGy of γ-ray irradiation.

### Example 2

33.75 g of PLGA (7525 DLG 2A), 28.9 g of NMP, 110 g of dichloromethane, 2.75 g of rotigotine, and 12.4 g of benzyl benzoate were precisely weighed, mixed, and dissolved, and then sterilized by filtration through a 0.22-µm filter. Thereafter, the dichloromethane was removed by rotary evaporation. Afterwards, the resultant was subpackaged into 1-mL pre-filled syringes in a sterile isolator.

### Example 3

33.75 g of PLGA (7525 DLG 2A), 28.9 g of NMP, 110 g of trichloromethane, 2.75 g of rotigotine, and 12.4 g of benzyl benzoate were precisely weighed, mixed, and dissolved, and then sterilized by filtration through a 0.22-µm filter. Thereafter, the trichloromethane was removed by rotary evaporation. Afterwards, the resultant was subpackaged into 1-mL pre-filled syringes in a sterile isolator.

### Example 4

33.75 g of PLGA (7525 DLG 2A), 28.9 g of NMP, 110 g of acetone, 2.75 g of rotigotine, and 12.4 g of benzyl benzoate were precisely weighed, mixed, and dissolved, and then sterilized by filtration through a 0.22-µm filter. Thereafter, the acetone was removed by rotary evaporation. Afterwards, the resultant was subpackaged into 1-mL pre-filled syringes in a sterile isolator.

### Example 5

67.5 g of PLGA (7525 DLG 2A), 57.8 g of NMP, 220 g of acetone, 5.5 g of rotigotine, and 24.8 g of benzyl benzoate were precisely weighed, mixed, and dissolved, and then sterilized by filtration through a 0.22-µm filter. Thereafter, the acetone was removed through a thin film evaporator at 40°C at a rate of 15 mL/min. Afterwards, the resultant was subpackaged into 1-mL pre-filled syringes in a sterile isolator.

### Example 6

33.75 g of PLGA (7525 DLG 2A), 110 g of acetone, 21.7 g of NMP, and 12.4 g of benzyl benzoate were precisely weighed, mixed, and dissolved, and then sterilized by filtration through a 0.22-µm filter. Subsequently, the acetone was removed by rotary evaporation to obtain a feed liquid A. 7.2 g of NMP and 2.75 g of rotigotine were weighed, mixed, and dissolved, and then sterilized by filtration through a 0.22-µm filter into the feed liquid A and mixed evenly. Afterwards, the resultant was subpackaged into 1-mL pre-filled syringes in a sterile isolator.

### Example 7 (Comparative Example)

33.75 g of PLGA (5050 DLG 2E), 38.8 g of NMP, and 6.5 g of sterile pramipexole dihydrochloride were precisely weighed, stirred until dispersed, and mixed well, and then subpackaged into 1-ml pre-filled syringes. Finally, they were sterilized by 25 kGy of γ-ray irradiation.

### Example 8

33.75 g of PLGA (5050 DLG 2E), 38.8 g of NMP, and 110 g of acetone were precisely weighed, mixed, and dissolved, and then filtered through a 0.22-µm filter membrane. Thereafter, the acetone was removed by rotary evaporation to obtain a feed liquid A. 6.5 g of sterile pramipexole dihydrochloride was added to the feed liquid A via a sterile feeding device (αβ valve), stirred until dispersed, and mixed well. Afterwards, the resultant was subpackaged into 1-mL pre-filled syringes in a sterile isolator.

### Experimental Results of Examples

### Experimental Example 1: Study on Solvent Residues

Approximate 0.1 to 0.5 g of each of samples in Examples 1 to 8 were precisely weighed, and placed in a headspace vial, and 0.5 to 5 mL of dimethyl sulfoxide and water were precisely added and sealed immediately to obtain a test solution. The above test solutions and the reference solutions were measured and detected by gas chromatography. The amount of the solvent residues was calculated by the peak area according to the external standard method. The results were detailed in Table 1.

**Table 1 Results of Solvent Residues**

| Example | Volatile Solvent | Solvent Evaporation Conditions | Volatile Solvent Residues (W/W) | Concentration Limit ^{[Note 1]} (W/W) |
|---|---|---|---|---|
| 2 | Dichloromethane | Rotary evaporation: 20°C×2min+40°C×2h | 0.002% | 0.06% |
| 3 | Trichloromethane | Rotary evaporation: 25°C×2min+45°C×3h | 0.005% | 0.006% |
| 4 | Acetone | Rotary evaporation: 24°C×2min+40°C×2h | 0.07% | 0.5% |
| | | Rotary evaporation: 24°C×2min+40°C×3h | 0.03% | |
| 5 | Acetone | Film evaporation: 40°C | 0.06% | |
| 6 | Acetone | Rotary evaporation: 24°C×2min; 40°C×3h | 0.03% | |
| 8 | Acetone | Rotary evaporation: 24°C×2min; 40°C×3h | 0.02% | |

| | | | | |
|---|---|---|---|---|
| ^{[Note 1]}: The concentration limit was subject to that specified in ICH Q3C, chp2020 Volume IV 0861, and the calculation basis was the total daily dose (a sum of all the active pharmaceutical ingredients and excipients in the formulation product). | | | | |

The results in Table 1 showed that all the volatile solvent residues in the samples of Examples 2 to 6 and Example 8 were in line with the limit standards and met the requirements for safety of formulations.

### Experimental Example 2: Study on In Vitro Release Characteristics of In-Situ Gel Formulation

### (1) Study on in vitro burst release of rotigotine-in-situ gel formulation

A 1-mL syringe connected with a 21G needle (outer diameter: 0.8 mm) was utilized to aspirate about 0.1 mL of samples, and precisely weighed. The samples were slowly injected dropwise into 50 mL of release media (0.01M phosphate buffer solution (0.2% SDS, adjusting the pH to 7.40)). The syringe was precisely weighed again. The injected amount of the samples was calculated by the decrement method. The sample vials were placed and held in a constant temperature oscillating water bath at 25±0.5°C. Triplicates were prepared for each sample. 2 mL of supernatant was collected at the predetermined points in time, while supplementing 2 mL of the release media at the same temperature. The above test solution was collected and tested by HPLC. The cumulative amount of release was calculated according to the external standard method. The test results of the *in vitro* release characteristics of rotigotine in Examples 1 to 6 were detailed in Table 2. The *in vitro* release characteristic was a mass percentage of the released drug to the total active pharmaceutical ingredient in the formulation.

### (2) Study on in vitro release characteristics of pramipexole-in-situ gel formulation

A 1-mL syringe connected with a 21G needle (outer diameter: 0.8 mm) was utilized to aspirate 0.1 mL of samples, and precisely weighed. The samples were slowly injected into 100 mL of release media (0.01M phosphate buffer solution (0.2% SDS, adjusting the pH to 7.40)). The syringe was precisely weighed again. The injected amount of the samples was calculated by the decrement method. The sample vials were placed in a constant temperature oscillating water bath at 37 ± 0.5°C and oscillated at 50 rpm. Triplicates were prepared for each sample. 2 mL of supernatant was collected at the predetermined points in time, while supplementing 2 mL of the release media at the same temperature. The above test solution was collected and tested by HPLC. The cumulative amount of release was calculated according to the external standard method. The test results of the *in vitro* release characteristics of pramipexole in Examples 7 and 8 were detailed in Table 3.

**Table 2 Test Results of In Vitro Release Characteristics of Rotigotine**

| | *In Vitro* Release Characteristics % (W/W) | | | | | |
|---|---|---|---|---|---|---|
| Time/h | Example 1 | Example 2 | Example 3 | Example 4 | Example 5 | Example 6 |
| 0.25h | 0.2% | 0.3% | 0.3% | 0.2% | 0.2% | 0.2% |
| 0.5h | 0.4% | 0.6% | 0.6% | 0.6% | 0.5% | 0.5% |
| 1h | 1.0% | 1.3% | 1.2% | 1.1% | 1.2% | 1.0% |
| 2h | 1.7% | 2.1% | 2.0% | 1.7% | 1.7% | 1.8% |
| 3h | 2.6% | 2.5% | 2.5% | 2.2% | 2.4% | 2.4% |
| 4h | 3.1% | 2.7% | 3.0% | 2.7% | 2.9% | 3.0% |
| 6h | 4.0% | 3.8% | 3.6% | 3.5% | 3.6% | 3.8% |
| 8h | 4.6% | 4.3% | 4.0% | 4.0% | 4.2% | 4.3% |
| 24h | 7.0% | 6.5% | 5.5% | 6.1% | 6.3% | 6.6% |
| 48h | 8.6% | 7.9% | 7.0% | 7.3% | 7.5% | 7.8% |

The results in Table 2 showed that there were no significant differences in 48h *in vitro* release characteristics in Examples 2 to 6 as compared to Example 1, indicating that the sterile rotigotine in-situ gels prepared by the solvent removal technique did not influence the release characteristics of the formulations.

**Table 3 Results of In Vitro Release Characteristics of Pramipexole**

| | *In Vitro* Release Characteristics % (W/W) | |
|---|---|---|
| Time/h | Example 7 | Example 8 |
| 1h | 10.6% | 11.2% |
| 4h | 13.6% | 13.8% |
| 8h | 14.4% | 14.9% |
| 48h | 15.4% | 16.2% |

The results in Table 3 showed that there were no significant differences in 48h *in vitro* release characteristics in Example 8 as compared to Example 7, indicating that the sterile pramipexole in-situ gel prepared by the solvent removal technique did not influence the release characteristics of the formulation.

### Experimental Example 3: Study on Related Substances in Rotigotine-In-Situ Gel Formulation

After being placed at 2 to 8°C under the dark condition for 6 months, 180 mg of the samples were weighed and placed in a 2-mL volumetric flask, to which 0.4 mL of stock solution of impurity as a reference substance was added, dissolved with a diluent and diluted to the scale. After shaking up, the content of the rotigotine-related substances was detected by HPLC. The test results of the contents of the related substances in Examples 1 to 4 and Example 6 were detailed in Table 4. The content of the related substance was the mass percentage of each related substance to the in-situ gel formulation.

**Table 4 Test Results of Contents of Rotigotine-Related Substances**

| Examples | | Example 1 | Example 2 | Example 3 | Example 4 | Example 6 |
|---|---|---|---|---|---|---|
| Impurity B | Desthienylethyl rotigotine | 0.51% | 0.09% | 0.10% | 0.09% | 0.09% |
| Impurity C | Despropyl rotigotine | 0.68% | Not Detected | Not Detected | Not Detected | Not Detected |
| Impurity D | Ethyl-rotigotine | Not Detected | Not Detected | Not Detected | Not Detected | Not Detected |
| Impurity E | Rotigotine N-Oxide | 0.13% | 0.09% | 0.12% | 0.06% | Not Detected |
| Impurity F | Acetyl-rotigotine | Not Detected | Not Detected | Not Detected | Not Detected | Not Detected |
| Impurity K | 7,8-Dihydronaphthol | 0.06% | Not Detected | Not Detected | Not Detected | Not Detected |
| Impurity G | N,N-Dithienylethyl rotigotine | Not Detected | Not Detected | Not Detected | Not Detected | Not Detected |
| Impurity H | Rotigotine methyl ether | Not Detected | Not Detected | Not Detected | Not Detected | Not Detected |
| Impurity I | Rotigotine p-toluene sulfonate | Not Detected | Not Detected | Not Detected | Not Detected | Not Detected |
| Impurity J | Rotigotine thienylethyl ether | 0.16% | 0.15% | 0.16% | 0.15% | 0.15% |
| Unspecified single largest impurity | | 2.20% | Not Detected | Not Detected | Not Detected | Not Detected |
| Total impurity content | | 4.58% | 0.33% | 0.38% | 0.30% | 0.24% |
| Total number of impurities | | 15 | 3 | 3 | 3 | 2 |

The results in Table 4 showed that after the formulation of Example 1 was sterilized by γ-ray irradiation, impurities B and C increased significantly, and unspecified single largest impurity appeared. Impurity E increased slightly in Examples 2 to 4 as compared to Example 6, while the other impurities did not change significantly. As could be appreciated from the results of the related substances, the impurities in the formulation were significantly increased after irradiation and did not meet the limit requirements for impurities, whereas the impurities in the formulations prepared by the solvent removal technique did not increase significantly and could meet the limit requirements for impurities.

### Experimental Example 4: Study on Plasma Concentrations of Rotigotine-In-Situ Gel Formulations in Rats

SD male rats (N=6) were selected to study the pharmacokinetics (PK) behavior *in vivo.* The dosage was 5 mg/kg (calculated as rotigotine). The formulations of some of the above Examples were injected subcutaneously. Blood was sampled from the tail vein at predetermined points in time. After the plasma samples were treated by Solid-Phase Extraction (SPE), the rotigotine concentration was determined by the LC-MS/MS method. The test results of the plasma concentrations of the formulations of Example 1 and Example 4 in rats were shown in Table 5, and the curves of plasma concentration vs. time were as shown in FIG. 1.

**Table 5 Test Results of Plasma Concentrations of Rotigotine-In-Situ Gel Formulations in Rats**

| Time (h) | Plasma Concentration (ng/mL) | |
|---|---|---|
| | Example 1 | Example 4 |
| 0 | 0.00 | 0.00 |
| 0.25 | 4.56 | 5.16 |
| 0.5 | 5.86 | 6.83 |
| 1 | 5.36 | 6.14 |
| 2 | 5.12 | 4.93 |
| 4 | 4.52 | 4.71 |
| 6 | 3.97 | 3.90 |
| 8 | 3.62 | 3.82 |
| 24 | 1.54 | 1.60 |
| 48 | 1.01 | 0.85 |
| 96 | 0.65 | 0.60 |
| 120 | 0.67 | 0.70 |
| 168 | 0.73 | 0.70 |
| 216 | 0.73 | 0.73 |
| 288 | 0.69 | 0.53 |
| 336 | 0.71 | 0.52 |

The results in Table 5 and FIG. 1 showed that the pharmacokinetics behaviors of the drugs in the formulations of Example 4 and Example 1 in rats were consistent, indicating that the sterile rotigotine-in-situ gels prepared by the solvent removal technique did not influence the *in vivo* release characteristics of the formulations.

## Claims

1. A preparation method of an in-situ gel formulation, the preparation method comprising the steps of:
i: mixing a biodegradable polymer and a volatile solvent among raw materials for preparation, optionally with part or all of components among the remaining raw materials for preparation, until homogeneous to obtain a feed liquid I;
ii: sterilizing the feed liquid I by filtration to obtain a sterile feed liquid I;
iii: removing the volatile solvent from the sterile feed liquid I; and
when the feed liquid I does not contain all of the raw materials for preparation, the preparation method further comprising the step of:
iv: mixing a product obtained in iii with the raw material(s) not contained in the feed liquid I which is(are) sterile until homogeneous under sterile conditions;
wherein an active pharmaceutical ingredient, a non-volatile solvent, and an optional release modifier are also among the raw materials for preparation.

2. The preparation method according to claim 1, wherein when the feed liquid I does not contain all of the raw materials for preparation, the product obtained in iii is mixed with the raw material(s) not contained in the feed liquid I until homogeneous in iv by any one or more of the following methods (a) to (g):
(a) adding the active pharmaceutical ingredient which is sterile through a sterile feeding device;
(b) adding a solution of the active pharmaceutical ingredient and the non-volatile solvent after filtration sterilization;
(c) adding a solution of the active pharmaceutical ingredient and the release modifier, if present, after filtration sterilization;
(d) adding a solution of the active pharmaceutical ingredient, the non-volatile solvent, and the release modifier, if present, after filtration sterilization;
(e) adding the non-volatile solvent after filtration sterilization;
(f) adding a solution of the non-volatile solvent and the release modifier, if present, after filtration sterilization; and
(g) adding the release modifier, if present, after filtration sterilization;
preferably, when the feed liquid I does not contain all of the raw materials for preparation, the feed liquid I satisfies any one of the following conditions (j) to (p):
(j) the feed liquid I contains the active pharmaceutical ingredient and the non-volatile solvent and does not contain the release modifier;
(k) the feed liquid I contains the active pharmaceutical ingredient and the release modifier and does not contain the non-volatile solvent;
(l) the feed liquid I contains the active pharmaceutical ingredient and does not contain the non-volatile solvent or the release modifier;
(m) the feed liquid I contains the non-volatile solvent and the release modifier and does not contain the active pharmaceutical ingredient;
(n) the feed liquid I contains the non-volatile solvent and does not contain the active pharmaceutical ingredient or the release modifier;
(o) the feed liquid I contains the release modifier and does not contain the active pharmaceutical ingredient or the non-volatile solvent; and
(p) the feed liquid I does not contain the active pharmaceutical ingredient, the non-volatile solvent, or the release modifier.

3. The preparation method according to claim 1 or 2, wherein the volatile solvent is a volatile alkane, a volatile halogenated alkane, a volatile alcohol, a volatile ketone, a volatile ester, a volatile organic cyanide, or a volatile ether;
preferably, among the raw materials for preparation, the mass ratio of the volatile solvent to the biodegradable polymer varies from 10:1 to 1:1.

4. The preparation method according to any one of claims 1 to 3, wherein the volatile solvent is dichloromethane, trichloromethane or acetone.

5. The preparation method according to any one of claims 1 to 4, wherein in the step iii, a way to remove the volatile solvent from the sterile feed liquid I is volatilization under one or more conditions selected from decompression, temperature control, ventilation, and stirring.

6. The preparation method according to any one of claims 1 to 5, wherein the biodegradable polymer is a polyester, a polyester copolymer, or a block copolymer.

7. The preparation method according to any one of claims 1 to 6, wherein the biodegradable polymer is PLGA, PLA, PCL, POE, PEG-PLA, PLA-PEG-PLA, PEG-PLGA, PEG-PLGA-PEG, TPGS-PLGA, or TEG-POE.

8. The preparation method according to any one of claims 1 to 7, wherein the non-volatile solvent is N-methyl pyrrolidone and/or dimethyl sulfoxide.

9. The preparation method according to any one of claims 1 to 8, wherein the optional release modifier is one or more selected from ethyl acetate, medium-chain triglyceride(s), glyceryl triacetate, glyceryl tricaprylate, benzyl benzoate, and benzyl alcohol;
preferably, the optional release modifier is benzyl benzoate and/or glyceryl triacetate.

10. An in-situ gel formulation obtained by the preparation method according to any one of claims 1 to 9.
